# EUROPEAN PATENT SPECIFICATION

(11) **EP 0 505 608 B1**
(45) Date of publication and mention of the grant of the patent: **11.08.1999**
(21) Application number: 91116311.1
(22) Date of filing: 25.09.1991
(51) Int. Cl.: A61K 31/48

(54) **Alpha-dihydroergocryptine pharmaceutical preparations with neuroprotective acitivity**
Alpha-Ergocryptine enthaltende pharmazeutische Zusammensetzungen mit nervenschützender Wirkung
Compositions pharmaceutiques à l'alpha-dihydro-ergocryptine avec une activité neuroprotectrice

(30) Priority: 28.03.1991 IT MI910843
(43) Date of publication of application: 30.09.1992
(73) Proprietor: POLICHEM S.A., Luxembourg (LU)
(72) Inventor: Poli, Stefano, I-20141 Milano (IT); Mailland, Federico, I-20141 Milano (IT); Coppi, Germano, I-20141 Milano (IT)
(74) Representative: Minoja, Fabrizio, Dr.

(56) References cited:
- GB-A- 2 173 101
- PROG. NEURO-PSYCHOPHARMACOL. & BIOL. PSYCHIAT., vol. 11, no. 1, 1986, pages 87-90, Pergamon Journals Ltd, GB; K.D. DATTA et al.: "Efficacy of oral hydergine (ergoloid mesylates) in alcohol related encephalopathy"
- J.E.F. REYNOLDS et al.: "Martindale, The Extra Pharmacopoeia", 29th edition, February 1989, pages 1051-1052, The Pharmaceutical Press, London, GB

## Description

The present invention relates to the use of α-dihydroergocryptine (hereinafter also named "α-DHEK") or a physiologicaly acceptable salt thereof as the only active ingredient for the preparation of a medicament useful for the protection of cerebral nerve cells from the degenerative effects caused by endogenous and exogenous toxic substances.

α-Dihydroergocryptine, or 12'-hydroxy-2'-(1-methylethyl)-5'α-(2-methylpropyl)-9,10-dihydroergotaman-3',6',18-trione, is a well-known compound, which derives from the hydrogenation of the double bond at the 9,10-position of natural alkaloid α-ergocryptine.

α-DHEK is mostly employed in combination with dihydroergocristine and dihydroergocornine in the therapeutic treatment of cerebrovascular disorders, especially in old people.

In fact, dihydroergocryptine, and generally other analogous hydrogenated alkaloids, are known to bind α and D receptors in central and peripheral nervous system.

According to said pharmacological activities, senile cerebrovascular insufficiency, in its several symptoms, is the main therapeutic indication for α-dihydroergocryptine, whether alone or in combination.

Italian Patent 1.200.603 (27.01.89), in the applicant's name, discloses pharmaceutical preparations containing α-dihydroergocryptine as the active ingredient and the use thereof in the treatment of Parkinson's disease, depression and headache.

Martindale The Extra Pharmacopoeia, 29th edition, p. 1051, defines co-dergocrine (containing a-DHEK) as a metabolic enhancer, anyway without giving an indication of which activity for what substance of the three forming co-dergocrine. This document directs the use of co-dergocrine to the treatment of senile dementia, Alzheimer, multi-infarct type or other cerebrovascular diseases, The treatment therein provided is symptomatic. In fact it is proposed for pathologies which are partially similar as to the symptoms, but have different etiology.

K.D Datta et al., Prog. Neuro-Psychopharmacol. & Biol. Psychiat. , vol. 11, n.1, 1986, 87-90, discloses the efficacy of oral hydergine in the treatment of symptoms deriving from the action of alcohol, showing an improvement in symptoms of depression, sleep disturbance and agitation. The authors acknowledge the vasodilating activity of hydergine .

It has now been found that α-dihydroergocryptine has a surprising cerebral neuroprotective activity. Particularly, it prevents the death of cerebral cells due to endogenous and exogenous toxic substances.

Primary cultures of cerebellar neurons were prepared from eight day old rats, as described by Nicoletti et al. (J. Neurosc. 6, 1905, 1986). The cells were trypsinized, treated with trypsin inhibitor and deoxyribonuclease, then they were suspended in basal Eagle's medium (BMA) containing foetal calf serum (10%), 25 mM KCl, 2 mM glutamine and gentamicine 50 µg/ml. The suspended cells (10⁶/ml) were distributed on Petri dishes, which were pretreated with poly-L-lysine (10 µg/ml) as taking root substrate. Citosine arabinofuranoside (Ara-C) was added to the cultures after 16-18 hours, to avoid the replication of the glial or endothelial cells. After 7-9 days of in vitro maturation (DIV), the primary cultures consisted in a highly homogeneous neuronal population, which was represented by granule layer cells (>90%), with a small number of GABAergic neurons (about 5%), and few glial and endothelial cells as contaminants. Cultured granule cells mature in the first week, then they reach the definitive fenotype within 9-11 days. Subsequently, they undergo degenerative phenomena mainly induced by the indiscriminate NMDA receptors activation, which is not balanced by an adequate intracellular energetic level.

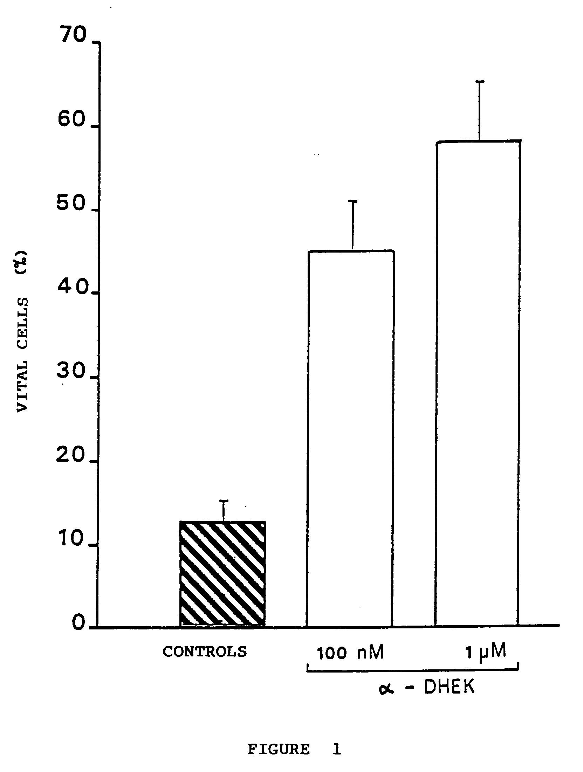

Cellular vitality was measured by labelling the cells with fluorescein diacetate and propidium iodide. Fluorescein reacts with vital cell esterases and gives a yellow-green colour under fluorescent light. Propidium iodide crosses the membranes of the degenerating cells and it binds to DNA, giving a red colour to the cells. Vitality was expressed as the percentage of fluorescein labelled cells and was evaluated from the analysis of four separate fields for each culture. α-DHEK is capable to protect cells from death at extremely low concentrations. Figure 1 shows the activity of α-DHEK, which was administered once a day for 13 days, on rat neuronal cell vitality on 13^{th} day.

Recent studies by Manev et al. (J. Pharm. Exp. Therap. 252, 419, 1990) have established that the slow degeneration of cultured neurons, induced by a single glutamic acid "pulse", is linked with an increase of Ca⁺⁺ basal influx, which lasts 20-50 min after glutamate removal from the incubation medium. On the basis of these results, neuronal degeneration was ascribed to Ca⁺⁺ homoeostasis alteration. Delayed Ca⁺⁺ influx was evaluated according to the following method. The cultures were washed three times with Locke's solution, containing 154 mM NaCl, 5.6 mM KCl, 5.6 mM glucose, 3.6 mM NaHCO₃, 2.3 mM CaCl₂ and 5 mM HEPES (pH 7.4). Mg⁺⁺ ions were omitted from the original Locke's solution. Glutamic acid was added at a 100 µM concentration for 15 minutes. At the end of the "pulse", the cells were washed three times with Locke's solution and incubated for 40 minutes at 37°C. Afterwards, the cultures were incubated with ⁴⁵Ca⁺⁺ (1 µC/dish) for 10 min. Radioactive Ca⁺⁺ uptake was stopped by washing the cultures with a chelating agent solution (154 mM choline chloride + 1 mM EGTA + 10 mM Tris-HCl pH 7.2) kept at 4°C. Intracellular radioactivity was evaluated after solubilizing the cells with 0.5 N NaOH (37°C for 30-60 min).

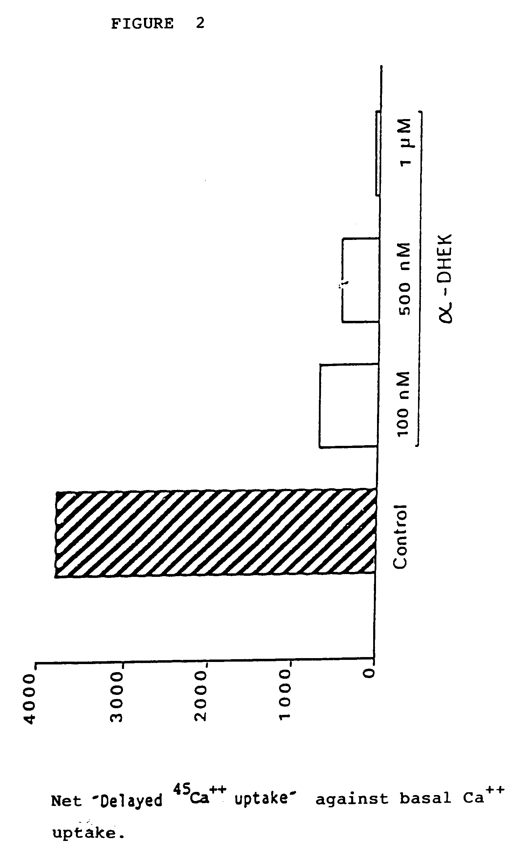

α-DHEK shows a good protection of the cells from delayed Ca⁺⁺ influx at extremely low concentrations. Figure 2 shows the activity of α-DHEK, administered once a day for 7 days, at various concentrations.

Systemic exposition to 1-methyl-4-phenyl-1,2,3,6-tetrahydopyridine (MPTP) causes in the man and in the monkey a Parkinson's disease-like syndrome. Low MPTP doses (1-5 mg/kg) in monkeys produce a selective loss of nervous cells in substantia nigra compacta, an irreversible dopamine decrease in the striatum and a remarkable extrapyramidal motor function decline. In rats, MPTP causes a cerebral damage of remarkable entity.

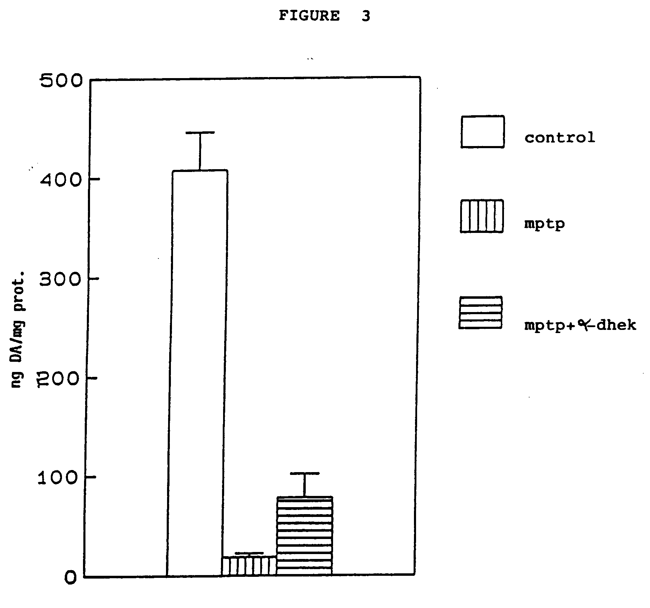

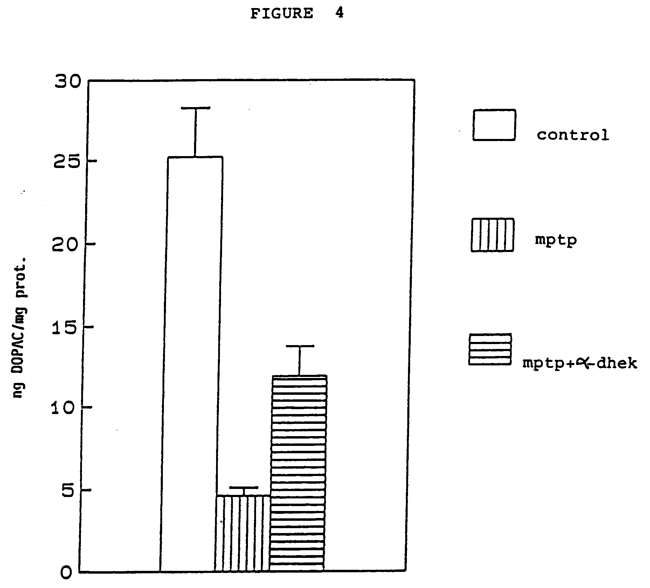

α-DHEK is capable of protecting mouse from MPTP toxic activity. MPTP administration to CB 57 mice causes a severe degeneration of nigro-striatal dopaminergic systems. MPTP doses (20 mg/Kg i.p.) were spaced at one hour each other. The animals were sacrificed 19 days after the treatment with MPTP. At this time, dopamine and DOPAC striatal levels were about 50% of those of the controls. α-Dihydroergocryptine administration (10 mg/Kg i.p.), given 30 minutes before the first MPTP dose, protects from the toxic agent degenerative effects. In the α-DHEK pretreated animals, dopamine and DOPAC levels were 3-4 times higher than those of the animals treated with MPTP only, as it is shown in Figures 3 and 4.

In Macaca fascicularis monkeys, mean weight 3 kg, neurotoxicity was induced by means of MPTP injections at the dose of 0.2 mg/kg i.v.; the eight animals were kept under anaesthesia with ketamine. A control group, consisting of 4 monkeys, was treated with saline only. MPTP treatment was carried out each day for 4 days in the eight animals; four of them received saline (4 ml/kg) orally (MPTP group) and the other four received α-dihydroergocryptine orally (MPTP + α-DHEK group) at the dose of 6 mg/kg (4 ml/kg) twice a day (each 12 hours) for four days. At the end of the treatment, the MPTP group monkeys were treated with saline once a day (4 ml/kg) for further 10 days and the MPTP+α-DHEK group ones were treated with α-dihydroergocryptine at the oral dose of 6 mg/kg (4 ml/kg), once a day for further 10 days. The control group was orally treated with saline (4 ml/kg) in the same way. The animals were observed from a behavioural point of view on the 14^{th} day; the 15^{th} day the animals were anaesthetized with ketamine, nembutal and pentobarbital, and their brains were withdrawn. Putamen and caudate were homogenized in 0.32 M sucrose and dopamine and homovanillic acid (HVA) were determined by HPLC method.

As it is shown in Table 1, α-dihydroergocryptine shows protective activity in the brain from MPTP neurotoxic action both in behavioural and neurochemical determinations.

**Table 1**

| **α-DHEK action on behaviour and on neurochemistry in MPTP neurointoxicated monkeys.** | | | |
|---|---|---|---|
| Treatments | Behaviour analysis (movements in 1 hour)* mean values | Caudate** | |
| | | dopamine (µg/g) | HVA (µg/g) |
| Control | 128.7 | 11.6 | 9.3 |
| MPTP | 38.9 | 2.7 | 1.8 |
| MPTP + α-DHEK | 87.9 | 5.9 | 4.7 |

| | | | |
|---|---|---|---|
| *Test carried out 1 hour after α-DHEK or saline oral administration. | | | |
| **Test carried out 24 hours after α-DHEK or saline oral administration; mean values. | | | |

Probably, MPTP exerts its neurotoxicity through two mechanisms. Dopamine metabolism, induced by monoaminoxydases (MAO), produces H₂O₂, which induces senescence of the dopaminergic neurons. MPTP releases dopamine from the pools and produces a remarkable increase in peroxydic radicals.

The other mechanism supposes that an intermediate in MPTP metabolism, produced by MAO, is a neurotoxic derivative.

α-Dihydroergocryptine, as such or as physiologically acceptable salt, can be administered by the oral, sublingual, parenteral or percutaneous routes, in form of pharmaceutical preparations properly formulated for the intended use.

To obtain neuroprotective activity, the daily dosage of α-DHEK, preferably expressed as methanesulfonate, can range from 0.5 to 100 mg, according to body weight and patient conditions.

The pharmaceutical compositions are prepared according to usual techniques, by using pharmaceutically acceptable excipients and carriers, and they may optionally contain a combination with other active ingredients with complementary or useful activities.

Examples of these preparations include capsules, pills, tablets, drops, ampoules for intramuscular or intravenous administration, oral sustained release forms, etc..

The following examples show the use of α-dihydroergocryptine in the preparation of a medicament in form of pharmaceutical preparations such as drops, ampoules and tablets.

| Drops | |
|---|---|
| 10 ml contain: | |
| α-Dihydroergocryptine methanesulfonate propylene glycol q.s. | 200 mg |

| Ampoules | |
|---|---|
| Each ampoule contains: | |
| α-Dihydroergocryptine methanesulfonate | 0.5 mg |
| propylene glycol | 100 mg |
| methanesulfonic acid | q.s. to pH 3 |
| bidistilled water | q.s. to ml 1 |

| Tablets | |
|---|---|
| each tablet contains: | |
| α-Dihydroergocryptine methanesulfonate | 20 mg |
| starch, lactose, magnesium stearate, microcrystalline cellulose, etc. | q.s. to 100 mg |

| Retard tablets | |
|---|---|
| each retard tablet contains: | |
| α-Dihydroergocryptine methanesulfonate | 40 mg |
| starch, lactose, magnesium stearate, microcrystalline cellulose, etc. | q.s. to 100 mg |

## Claims

1. The use of α-dihydroergocryptine or of a physiologically acceptable salt thereof as the only active ingredient for the preparation of a medicament for the protection of cerebral nerve cell from the degenerative effects caused by endogenous and exogenous toxic substances.

2. The use according to claim 1, wherein said α-dihydroergocryptine salt is the methanesulfonate salt.

3. The use according to claims 1-2, wherein said α-dihydroergocryptine is contained in the medicament in unitary dosages suitable for its admnistration from 0.5 to 100 mg/day.

4. The use according to claims 1-3, wherein said medicament is in form of pharmaceutical preparations suitable for the oral, sublingual, parenteral, percutaneous, and nasal administrations.

5. The use according to claim 4, wherein said pharmaceutical preparations include tablets, pills, drops, oral sustained release forms, forms for the endonasal administration, ampoules for the parenteral, intravenous and intramuscular administrations.

## Patentansprüche

1. Die Verwendung von α-Dihydroergocryptin oder eines physiologisch verträglichen Salzes davon als der einzige aktive Bestandteil für die Herstellung eines Medikaments für den Schutz von Gehirnnervenzellen vor degenerativen Wirkungen, verursacht durch endogene und exogene giftige Substanzen.

2. Die Verwendung nach Anspruch 1, bei welcher das α-Dihydroergocryptin-Salz das Methansulfonat-Salz ist.

3. Die Verwendung nach den Ansprüchen 1 und 2, bei welcher das α-Dihydroergocryptin in dem Medikament in Einheitsdosierungen enthalten ist, welche für die Verabreichung von 0.5 bis 100 mg/Tag geeignet sind.

4. Die Verwendung nach den Ansprüchen 1 bis 3, bei welcher das Medikament in der Form pharmazeutischer Präparate ist, die für die orale, sublinguale, parenterale, perkutane und nasale Verabreichung geeignet sind.

5. Die Verwendung nach Anspruch 4, bei welcher die pharmazeutischen Präparate Tabletten, Pillen, Tropfen, oral unterstützte Beigabeformen, Formen für die endonasale Verabreichung, Ampullen für die parenterale, intravenöse und intramuskuläre Verabreichung umfassen.

## Revendications

1. L'utilisation de la α-dihydro-ergocryptine, ou d'un sel physiologiquement acceptable de celle-ci, en tant que seul ingrédient actif pour la préparation d'un médicament destiné à la protection des cellules nerveuses cérébrales des effets dégénératifs causés par des substances toxiques endogènes et exogènes.

2. L'utilisation selon la revendication 1, dans laquelle ledit sel de α-dihydro-ergocryptine est le méthane-sulfonate.

3. L'utilisation selon les revendications 1 et 2, dans laquelle ladite α-dihydro-ergocryptine est contenue dans le médicament en doses unitaires convenant pour son administration à raison de 0,5 à 100 mg/jour.

4. L'utilisation selon les revendications 1 à 3, dans laquelle ledit médicament est sous la forme de préparations pharmaceutiques adaptées aux administrations orale, sublinguale, parentérale, percutanée et nasale.

5. L'utilisation selon la revendication 4, dans laquelle lesdites préparations pharmaceutiques comprennent des comprimés, pilules, gouttes, formes à libération prolongée pour l'administration orale, formes pour l'administration endonasale, ampoules pour les administrations parentérale, intraveineuse et intramusculaire.
